Europäisches Patentamt

European Patent Office

Office européen des brevets

(19)

(11) Veröffentlichungsnummer: **0 182 960 B1**

# (12) EUROPÄISCHE PATENTSCHRIFT

(45) Veröffentlichungstag der Patentschrift: **18.12.91**

(51) Int. Cl.⁵: **G01B 17/00,** D01H 13/26, G01N 33/36, B65H 63/06

(21) Anmeldenummer: **85105774.5**

(22) Anmeldetag: **10.05.85**

(54) **Vorrichtung zur mindestens näherungsweisen Bestimmung des Querschnittes von langgestreckten Gebilden.**

(30) Priorität: **26.10.84 CH 5160/84**

(43) Veröffentlichungstag der Anmeldung:
**04.06.86 Patentblatt 86/23**

(45) Bekanntmachung des Hinweises auf die
Patenterteilung:
**18.12.91 Patentblatt 91/51**

(84) Benannte Vertragsstaaten:
**DE FR GB IT**

(56) Entgegenhaltungen:
**CH-A- 515 487**
**CH-A- 543 075**
**DE-U- 2 116 782**
**US-A- 2 538 444**
**US-A- 3 750 461**

(73) Patentinhaber: **ZELLWEGER USTER AG**
**Wilstrasse 11**
**CH-8610 Uster(CH)**

(72) Erfinder: **Felix, Ernst**
**Bahnstrasse 35**
**CH-8610 Uster(CH)**

(74) Vertreter: **Dipl.-Phys.Dr. Manitz Dipl.-Ing.,**
**Dipl.-W.-Ing. Finsterwald Dipl.-Ing. Grämkow**
**Dipl.-Chem.Dr. Heyn Dipl.-Phys. Rotermund**
**Morgan, B.Sc.(Phys.) Robert-Koch-Strasse 1**
**W-8000 München 22(DE)**

## Beschreibung

Die Erfindung betrifft eine Vorrichtung zur mindestens näherungsweisen Bestimmung des Querschnitts von langgestrecktem Prüfgut, insbesondere von textilen Garnen, Vorgarnen und Bändern, sowie von Kabeln oder Drähten, mit einem Schallraum, durch welchen das Prüfgut geführt ist, mit mindestens einem an einer Wandung des Schallraums vorgesehenen Sender und mindestens einem Empfänger zur Erzeugung beziehungsweise zum Empfang stehender Schallwellen, und mit Mitteln zur Bestimmung des Einflusses des Prüfgut auf die Ausbreitung der Schallwellen, wobei bei der Messung das Prüfgut im Schallraum so positioniert ist, daß es wenigstens angenähert in einem Druckmaximum einer ersten und in einem Druckminimum einer zweiten Schallwelle zu liegen kommt.

Bei einer in der CH-A-543 075 beschriebenen Vorrichtung dieser Art sind die den oder die Schallsender und Schallempfänger tragenden Wandungen durch ebene Flächen gebildet, deren den Schallraum bildender Zwischenraum mit der freien Atmosphäre kommuniziert. Dieser Kontakt mit der freien Atmosphäre macht es unmöglich, diese bekannten Vorrichtungen an solchen Spinnereimaschinen zu verwenden, bei denen das Prüfgut mittels Druckluft durch Kanäle transportiert wird und der Querschnitt des Prüfguts im Bereich eines solchen Kanals bestimmt werden soll. Ein weiterer Nachteil dieser offenen Meßorgane besteht darin, daß die die Sender und Empfänger bildenden elektro-akustischen Wandler äußeren, die Meßresultate verfälschenden Einflüssen ausgesetzt sind.

Die ersten und zweiten Schallwellen werden bei der bekannten Vorrichtung entweder gleichzeitig an zwei zueinander parallelen Sender-/Empfänger-Paaren oder intermittierend an einem einzigen Paar erzeugt und weisen zur Ermöglichung einer umfassenden Signalauswertung zwei Frequenzen auf. Damit nun das Prüfgut in einem Schwingungsbauch der einen und in einem Schwingungsknoten der anderen Frequenz positioniert ist, müssen die zwei Frequenzen in einem bestimmten Verhältnis stehen. Es hat sich in der Praxis gezeigt, daß diese Forderung eine unerwünschte Einschränkung darstellt.

In der US-A-2 538 444 ist eine Vorrichtung zur Bestimmung des Querschnitts von textilem Prüfgut unter Verwendung von Schallwellen beschrieben. Diese Vorrichtung weist eine erste, eine Schallquelle beinhaltende Schallkammer und eine zweite, einen Schallempfänger beinhaltende Schallkammer auf, die über ein enges, das Prüfgut führende Rohr verbunden sind. Zur Ermittlung des Durchmessers des Prüfgutes wird die in Form von nicht stehenden Wellen über das enge Rohr übertragene Schallenergie gemessen.

Die Aufgabe der Erfindung besteht darin, mit stehenden Schallwellen arbeitende Vorrichtungen dahingehend zu verbessern, daß einerseits bei der Wahl der Frequenzen der stehenden Wellen mehr Freiheiten bestehen und andererseits eine Vorrichtung der eingangs beschriebenen Art in der Weise auszubilden, daß sie universell an allen Arten von Spinnereimaschinen und insbesondere an Spinnereimaschinen, bei denen das Prüfgut mittels Druckluft transportiert wird, eingesetzt werden kann und zudem eine optimale Abschirmung der Meßvorrichtung gegen äußere Einflüsse erreicht wird.

Diese Aufgabe wird bei einer Vorrichtung der eingangs genannten Art dadurch gelöst, daß der Schallraum mit Ausnahme von Durchlaßöffnungen für das Prüfgut allseits geschlossen ist, und daß Sender und Empfänger so an den Wandungen angeordnet sind, daß das Prüfgut bei der Messung von einander kreuzenden Schallwellen beaufschlagt ist.

Anhand der Beschreibung und der Zeichnungen werden nun Ausführungsbeispiele der Erfindung näher erläutert; dabei zeigt:

Fig. 1       die prinzipielle Anordnung eines Faserstranges in einem geschlossenen akustischen Resonator,

Fig. 2       die Ausbildung eines Resonators zur Kompensation von Vibrationen,

Fig. 3       einen Faserstrang in einem in verschiedenen Achsen angeregten akustischen Resonator,

Fig. 4       eine andere Ausführungsform eines akustischen Resonators,

Fig. 5 und 6       Ausführungen eines Resonators zur Kompensation von Vibrationen,

Fig. 7       einen Resonator analog Fig. 5 mit aussermittig geführtem Prüfgut.

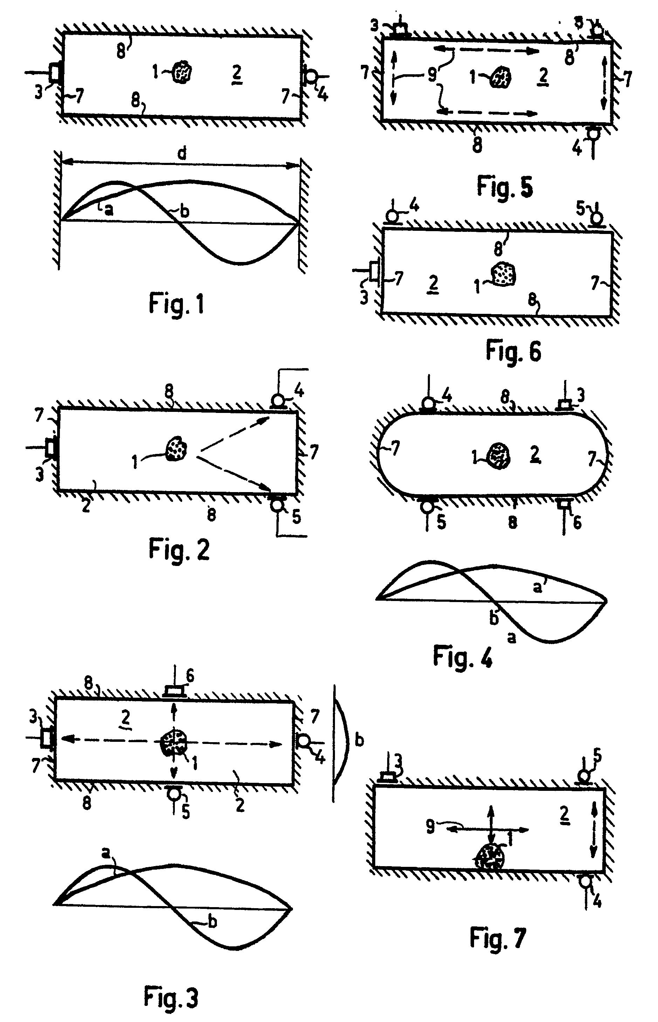

Für die Anordnung nach Fig. 1 als solche wird kein Patentschutz beansprucht.

In Fig. 1 stelle 2 den in Querrichtung allseits geschlossenen Schallraum dar, durch den das Prüfgut 1, das die Form eines Faserstranges, eines Kabels oder eines Drahtes aufweisen kann, senkrecht zur Zeichenebene hindurchbewegt wird. Selbstverständlich weisen die vordere und hintere Schallraumabdeckung, die parallel zur Zeichenebene liegen, entsprechende Durchlassöffnungen für das Prüfgut auf. Diese sind jedoch für die Bildung stehender Wellen im Schallraum nicht massgebend.

In einander gegenüberliegenden Begrenzungsflächen 7 des Schallraumes 2 ist eine Schallquelle 3, beispielsweise ein Lautsprecher und ein Schallempfänger, beispielsweise in Mikrofon eingebaut.

Wird die Schallquelle 3 mit einer Tonfrequenz angeregt, deren Frequenz bzw. Wellenlänge λ (in Luft) in einem bestimmten Verhältnis zur Distanz d zwischen den Begrenzungsflächen steht, bilden sich im Schallraum stehende Wellen. Beträgt die Wellenlänge λ das zweifache des Abstandes d, entsteht an den Begrenzungsflächen ein Schwingungskroten und in der Mitte (bei d/2) ein Schwingungsbauch (Kurve a). Wird die Frequenz verdoppelt (d.h. die Wellenlänge λ halbiert), sodass λ = d ist, verläuft die stehende Welle von Kurve b mit Schwingungsknoten bei 0, d/2 und d, und mit Schwingungsbäuchen bei 1/4d und 3/4d. Das Prüfgut verändert in an sich bekannter Weise das am Empfänger 4 eintreffende Signal, so dass aus der Veränderung gegenüber einem ungestörten Signal durch Auswertung der Amplituden- und/oder Phasen- und/oder Laufzeit-Unterschiede die Menge des Prüfgutes bestimmt werden kann.

Wie bereits in der CH-PS 543.075 dargelegt, werden für eine umfassende Signalauswertung mindestens zwei Frequenzen benötigt, um das Prüfgut 1 sowohl in einem Schwingungsbauch der einen Frequenz als auch in einem Schwingungsknoten der anderen Frequenz plazieren zu können. Dadurch ist es vorteilhaft, die Frequenzen in mindestens näherungsweise ganzzahligen Verhältnissen zu wählen.

Hierzu zeigt Figur 3 ein Beispiel. Dabei wirken die einander gegenüberliegenden Schallsender 3 und Schallempfänger 4 bzw. Schallsender 6 und Schallempfänger 5 zusammen, die in je zwei gegenüberliegenden Flächen 7, 8 des Schallraumes 2 angeordnet sind. Schallsender 3 regt eine stehende Welle a und Schallsender 6 eine dazu senkrecht orientierte stehendeWelle b an. Das zentral geführte Prüfgut 1 befindet sind dabei in einem Druckminimum (Geschwindigkeitsmaximum) der Welle b und in einem Druckmaximum (Geschwindigkeitsminimum) der Welle a.

Wie einleitend erwähnt, ist die Arbeit mit zwei Frequenzen vorteilhaft zur Kompensation von Störungen, wie beispielsweise Temperatureinflüsse und Schmutzablagerungen. Ist die Temperaturverteilung im Schallraum homogen, so ist die Kompensation des Temperatureinflusses möglich, wenn die Schwingungsrichtungen mit den beiden Frequenzen verschieden sind. Anders verhält es sich z.B. mit Schmutzablagerungen an den Wänden des Schallraumes. Solche beeinflussen die Schwingungen mit den beiden Frequenzen nicht mehr gleichartig. Weisen die Schwingungen die gleiche Richtung auf, so beeinflussen eventuelle Schmutzablagerungen an den Wänden die beiden Frequenzen eher gleichartig.

Der Schallraum 2 muss nicht unbedingt einen rechteckigen Querschnitt aufweisen. Er kann auch - wie dies in Fig. 4 gezeigt ist - eine andere Querschnittsform annehmen. Dies kann beispielsweise aus Herstellungsgründen vorteilhaft sein. Gleichzeitig zeigt Fig. 4 auch eine andere Anordnung von Sendern 3, 6 und Empfängern 4, 5. Sender 3, 6 können hierbei parallelgeschaltet sein und derart angeregt werden, dass eine Grundschwingung a entsteht. Gleichzeitig kann dieser Grundschwingung noch eine zweite Frequenz überlagert sein, so dass eine Oberwelle b entsteht. Die Empfänger 4, 5 können parallelgeschaltet sein und nehmen sowohl das Drucksignal der Grundwelle a in Gegenphase (180°) zum Sendersignal, als auch die erste Oberwelle b gleichphasig (d.h. mit 2 x 180° Phasenverschiebung) auf.

Wenn solche elektro-akustischen Messorgane auf Produktionsmaschinen angeordnet werden, erleiden sie durch die Maschinenvibrationen Erschütterungen. Es gibt praktisch keine akustischen Empfänger, die nicht auf Erschütterungen reagieren. Dabei ist ihre Empfindlichkeit gegenüber Erschütterungen vor allem in senkrechter Richtung zur Wandlermembran erheblich. Im Ausführungsbeispiel gemäss Fig. 2 sind nun zwei Schallempfänger 4, 5 gegenüberliegend angeordnet. Wenn diese Empfänger gleichnamig gepolt und parallelgeschaltet werden, heben sich die durch mechanische Erschütterungen ausgelösten Signale auf, während die Schallwellen der Eigenschwingungen in der längeren Querrichtung in Mitphase auftreffen und sich dabei addieren.

Störungen können auch durch Lärm ausserhalb des Messorgans erzeugt werden. Durch entsprechende Anordnung der Mikrofone sind auch hier Kompensationen möglich. Fig. 6 zeigt ein entsprechendes Beispiel. Die Mikrofone 4, 5 sind praktisch an den sich gegenüberliegenden Seiten des Resonators angeordnet. Beim Arbeiten auf der Grundwelle liegen daher die Druckmaxima gegenphasig an den Mikrofonen 4, 5. Sie können daher auch gegenphasig zusammengeschaltet werden. Lärm von aussen tritt aber eher gleichphasig an die beiden Mikrofone heran. Da diese aber gegenphasig gepolt sind, nehmen sie diesen störenden Schall nicht auf. Je nach dem Anwendungsfall ist die Anordnung der Mikrofone so zu wählen, dass störende Vibrationen bzw. Lärm optimal kompensiert werden.

Gemäss der in CH-PS 543.075 erläuterten Anordnung mit einander gegenüberliegenden Schallwänden, die nach oben und unten offen sind, können nur stehende Wellen erzeugt werden, wenn ihre halben Wellenlängen ein ganzzahliges Verhältnis des Abstandes zwischen Schallquelle und Schallempfänger beträgt. Im vorliegenden Verfahren mit allseitig geschlossenem Schallraum bestehen noch weitere Möglichkeiten, innerhalb des Schallraumes bestimmte Schwingungszustände, sogenannte Moden, anzuregen. Die Bildung solcher Moden ist an sich bekannt und beispielsweise in Philip M. Morse and K. Uno Ingard, Theoretical Acoustics, S. 467 - 522 und 554 - 576 (McGraw Hill, 1968); Bergmann-Schaefer, Lehrbuch der Experimentalphysik, Bd. I, S. 492 - 504 (Walter de Gruyter & Co., 1974) offenbart.

Die Aufgabe besteht nun darin, durch geeignete Wahl der Abmessungen des Schallraumes und der von den den Schallsendern 3, 6 abgestrahlten Frequenzen einen solchen Mode anzuregen, der in Bezug auf das Prüfgut 1 den grössten Einfluss auf das vom Schallempfänger 4 bzw. den Schallempfängern 4, 5 aufgenommene Signal ergibt.

Eine mögliche Anordnung zeigt Fig. 5. Der Schallsender 3 erzeugt einen sogenannten gemischten Schwingungsmode, dessen Schwingungsrichtung durch die Pfeile 9 angedeutet sind. Am Schallempfänger 4 entsteht ein Signal in Phase mit dem Sendesignal. Das Prüfgut 1 liegt dabei in einem Druck- und Geschwindigkeitsminimum, es beeinflusst daher die Eigenfrequenz des Modes praktisch nicht. Ein weiterer Schallempfänger 5 liegt ebenfalls in einem Druckminimum und liefert praktisch kein Signal. Der Schallsender 3 kann nun derart betrieben werden, dass auch die erste Oberwelle in der längeren Querrichtung entsteht. Dabei liegt das Prüfgut 1 im Druckmaximum (oder Geschwindigkeitsminimum) dieser Welle. Der Schallempfänger 5 ist nur auf das Signal dieses Schwingungszustandes empfindlich.

Fig. 7 zeigt eine Ausbildung des Resonators gemäss Fig. 3 oder 1, bei dem das Prüfgut 1 längs einer Begrenzungsfläche 8 des Schallraumes 2 verläuft und dabei diese berührt. In der Praxis ist dies die normale Prüfgutanordnung, da es kaum möglich ist, das Prüfgut ohne unzulässig grosse Spannung in Schallraum-Mitte schwebend durchzuziehen. Für die Beeinflussung des Schallfeldes gelten dabei die gleichen Ueberlegungen wie vorstehend bei der Erläuterung der Fig. 5.

**Patentansprüche**

1. Vorrichtung zur mindestens näherungsweisen Messung des Querschnittes von langgestrecktem Prüfgut, insbesondere von textilen Garnen, Vorgarnen und Bändern, sowie von Kabeln oder Drähten, mit einem Schallraum (2) durch welchen das Prüfgut (1) geführt ist, mit mindestens einem an einer Wandung (7, 8) des Schallraumes (2) vorgesehenen Sender (3, 6) und mindestens einem Empfänger (4, 5) zur Erzeugung bzw. zum Empfang stehender Schallwellen und mit Mitteln zur Bestimmung des Einflusses des Prüfgutes (1) auf die Ausbreitung der Schallwellen, wobei bei der Messung das Prüfgut im Schallraum (2) so positioniert ist, daß es wenigstens angenähert in einem Druckmaximum einer ersten und einem Druckminimum einer zweiten Schallwelle zu liegen kommt, dadurch **gekennzeichnet,** daß der Schallraum (2) mit Ausnahme von Durchlaßöffnungen für das Prüfgut (1) allseits geschlossen ist, und daß Sender (3, 6) und Empfänger (4, 5) so an den Wandungen (7, 8) angeordnet sind, daß das Prüfgut (1) bei der Messung von einander kreuzenden Schallwellen beaufschlagt ist.

2. Vorrichtung nach Anspruch 1, **gekennzeichnet,** durch je zwei Sender (3, 6) und Empfänger (4, 5), welche so angeordnet sind, daß die Schallwellen bei der Messung einander senkrecht kreuzen.

3. Vorrichtung nach Anspruch 1, dadurch **gekennzeichnet,** daß an einer ersten Wandung (8) des Schallraumes (2) ein Sender (3) und ein erster Empfänger (5) im gegenseitigen Abstand angeordnet sind, und daß an einer der ersten der Wandung gegenüberliegenden zweiten Wandung (8) ein zweiter Empfänger (4) vorgesehen ist.

4. Vorrichtung nach Anspruch 1, dadurch **gekennzeichnet,** daß an zwei einander gegenüberliegenden Wandungen (8) des Schallraums (2) je ein Sender (3, 6) und ein Empfänger (4, 5) im gegenseitigen Abstand angeordnet sind.

5. Vorrichtung nach Anspruch 1, dadurch **gekennzeichnet,** daß an einer ersten Wandung (8) des Schallraums (2) zwei Empfänger (4, 5) im gegenseitigen Abstand angeordnet sind, und daß an einer quer zu dieser ersten Wandung verlaufenden zweiten Wandung (7) ein Sender (3) vorgesehen ist.

**Claims**

1. Device for measuring at least approximately the cross-section of an elongated product, in particular of textile yarns, roving and bands, as well as of cables or wires, comprising an acoustic chamber (2) through which the product (1) to be measured is guided, at least one transmitter (3, 6) arranged on a wall (7, 8) of the acoustic chamber (2) and at least one receiver (4, 5) for generating or receiving stationary acoustic waves, and comprising means for determining the influence of the measured product (1) on the propagation of the acoustic waves, wherein the measured product is positioned during the measurement in the acoustic chamber (2) in such a manner that it passes at least approximately by a pressure maximum of a first acoustic wave and a pressure minimum of a second acoustic wave, characterised in that the acoustic chamber (2) is closed on all sides, with the exception of passage openings for the measured product (1); and in that the transmitter (3, 6) and the receiver (4, 5) are arranged on the walls (7, 8) in such a manner that during the measurement, the measured product (1) is impinged on by mutually crossing acoustic waves.

2. Device according to claim 1, characterised by two transmitters (3, 6) and two receivers (4, 5) which are arranged in such a manner that during the measurement the acoustic waves cross each other at right angles.

3. Device according to claim 1, characterised in that one transmitter (3) and a first receiver (5) are arranged spaced from each other on a first wall (8) of the acoustic chamber (2); and in that a second receiver (4) is arranged on a second wall opposite to the first wall.

4. Device according to claim 1, characterised in that one transmitter (3, 6) and one receiver (4, 5) are arranged spaced from each other in each case on two mutually opposed walls (8) of the acoustic chamber (2).

5. Device according to claim 1, characterised in that two receivers (4, 5) are arranged spaced from each other on a first wall (8) of the acoustic chamber (2); and in that one transmitter (3) is arranged on a second wall (7) extending transversely to this first wall.

**Revendications**

1. Dispositif pour mesurer au moins approximativement la section transversale d'un produit allongé, en particulier de filés, de mèches et de bandes textiles, ainsi que de câbles ou de fils, comprenant une chambre acoustique (2) à travers laquelle le produit à mesurer (1) est guidé, au moins un émetteur prévu sur l'une des parois (7, 8) de la chambre acoustique (2) et au moins un récepteur (4, 5) pour produire ou recevoir des ondes sonores stationnaires, et comprenant des moyens pour déterminer l'influence du produit à mesurer (1) sur la propagation des ondes sonores, le produit à mesurer étant, lors de la mesure, positionné dans la chambre acoustique (2) de telle manière qu'il passe au moins approximativement par un maximum de pression d'une première onde sonore et par un minimum de pression d'une seconde onde sonore, caractérisé en ce que la chambre acoustique (2) est fermée de tous côtés à l'exception d'ouvertures de passage pour le produit à mesurer (1), et en ce que l'émetteur (3, 6) et le récepteur (4, 5) sont agencés sur les parois de telle manière que le produit à mesurer soit atteint lors de la mesure par des ondes sonores qui se croisent mutuellement.

2. Dispositif selon la revendication 1, caractérisé en ce qu'il comprend deux émetteurs (3, 6) et deux récepteurs (4, 5) qui sont agencés de telle manière que lors de la mesure les ondes sonores se croisent à la perpendiculaire.

3. Dispositif selon la revendication 1, caractérisé en ce qu'un émetteur (3) et un premier récepteur (5) sont agencés à distance l'un de l'autre sur une première paroi (8), et en ce qu'un second récepteur (4) est prévu sur une seconde paroi (8) opposée à la première paroi.

4. Dispositif selon la revendication 1, caractérisé en ce qu'un émetteur (3, 6) et un récepteur (4, 5) sont agencés à distance l'un de l'autre sur deux parois (8) opposées l'une à l'autre de la chambre acoustique (2).

5. Dispositif selon la revendication 1, caractérisé en ce que deux récepteurs (4, 5) sont agencés à distance l'un de l'autre sur une première paroi de la chambre acoustique (2), et en ce qu'un émetteur est prévu sur une seconde paroi (7) s'étendant transversalement à cette première paroi.

Fig. 1

Fig. 2

Fig. 3

Fig. 4

Fig. 5

Fig. 6

Fig. 7